Europäisches Patentamt

European Patent Office

Office européen des brevets

(11) Publication number: 0 324 157
A1

# EUROPEAN PATENT APPLICATION

(21) Application number: 88121602.2

(22) Date of filing: 23.12.88

(51) Int. Cl.⁴: C12N 15/00 , A61K 39/205 , C12P 21/00

The applicant has filed a statement in accordance with Rule 28 (4) EPC (issue of a sample only to an expert). Accession number(s) of the deposit(s): FERM BP-1631

A request for correction of deposition number "FERM-1613" (must be "FERM-1631") has been filed pursuant to Rule 88 EPC. A decision on the request will be taken during the proceedings before the Examining Division (Guidelines for Examination in the EPO, A-V, 2.2).

(30) Priority: 26.12.87 JP 330896/87

(43) Date of publication of application:
19.07.89 Bulletin 89/29

(84) Designated Contracting States:
AT BE CH DE ES FR GB GR IT LI LU NL SE

(71) Applicant: Juridical Foundation The Chemo-Sero-Therapeutic Research Institute 668, Okubo Shimizu-machi Kumamoto-shi Kumamoto-ken(JP)

(72) Inventor: Sakamoto, Shinichi
No. 2033-11, Suya Nishi-Goshi-machi Kikuchi-gun Kumamoto-ken(JP)
Inventor: Ide, Toshio
No. 2738-64, Suya Nishi-Goshi-machi Kikuchi-gun Kumamoto-ken(JP)
Inventor: Tokiyoshi, Sachio
No. 14-19, Wakaba 3-chome Kumamoto-shi Kumamoto-ken(JP)
Inventor: Yamamoto, Michitaka
No. 2127-20, Sakae Nishi-Goshi-machi Kikuchi-gun Kumamoto-ken(JP)

(74) Representative: Vossius & Partner
Siebertstrasse 4 P.O. Box 86 07 67
D-8000 München 86(DE)

(54) Gene fragment coding for a rabies virus glycoprotein and process for producing a rabies virus glycoprotein using the same.

(57) A gene fragment is described which encodes a rabies virus glycoprotein. Furthermore, a recombinant rabies virus glycoprotein expressed by using said gene fragment which is extremely useful for preparing rabies vaccines and diagnostic reagents and a process for preparing a rabies virus glycoprotein are described which process comprises introducing into eukaryotic cells a shuttle vector wherein the gene fragment coding for a rabies virus glycoprotein is inserted downstream of a promoter capable of functioning in eukaryotic cells, and culturing said transformed cells to yield a rabies virus glycoprotein in the cytoplasm of said cells. Since the G protein can be expressed in the cytoplasm as a granule, not coupled to the cell membrane, the process of this invention can give the desired G protein in high yields and high purity. The G protein of this invention can not only react with antibodies against the G protein but also induces antibodies having an activity of neutralizing rabies virus when injected into animals.

## GENE FRAGMENT CODING FOR A RABIES VIRUS GLYCOPROTEIN AND PROCESS FOR PRODUCING A RABIES VIRUS GLYCOPROTEIN USING THE SAME

The present invention relates to a rabies virus glycoprotein useful for preparing rabies vaccines or other diagnostic reagents. More particularly, the present invention relates to a gene fragment coding for a rabies virus glycoprotein, a recombinant rabies virus glycoprotein prepared by expressing said gene fragment and a process for producing said rabies virus glycoprotein.

Rabies, one of the oldest diseases known to human, is a quite dangerous infectious disease common to humans and other mammals which may lead to 100 % death in all mammals when affected with this disease.

In Japan, it is favored by such a good condition that it is an island country and further Rabies Prevention Act has been enacted for dogs, which are main carriers of said disease to human, and preventive injections have been conducted every year. Consequently, this disease has successfully been eradicated in 1957. Ever since over more than a quarter century, Japan occupies a very high place in the world as regards rabies eradication, i.e. no occurrence of said disease. However, Japan is rather a rare case where rabies is eradicated and it is still prevalent in the world. According to statistics by WHO in 1982, rabies is much prevailed in Vietnam, Thailand, the Philippins, India, Brazil, Ecuador and the like and it is presumed that there are up to 50,000 deaths due to rabies a year even in one country of India. Also in Japan, the problem of rabies does not completely vanish but vaccination for rabies is still regarded as an important protective innoculation for passengers abroad especially Southeast Asia.

A historical review of the rabies vaccine reveals that the rabies vaccine has been developed starting from attenuated vaccines by Pasteur Institute in France to innactivated vaccines prepared from mammalian brain or partially purified vaccines thereof and further to vaccines prepared by cell culture employing mammalian or avian culture cells. However, all of these vaccines are the first generation vaccines and still impose problems concerning safety, productivity and the like.

The Nishigahara strain (derivative) is a virus strain suitable for preparing a vaccine for both animal and human which has greatly contributed to eradicate rabies in recent Japan. This virus strain has been prepared by modifying Pasteur fixed rabies virus prepared by Pasteur Institute in France. That is, since the Pasteur fixed rabies virus had a latent period of 5 to 6 days in a rabbit passage, Kondo et al. in Japan Ministry of Agriculture and Forestry, Nishigahara Animal Plague Research Institute have cultivated the virus for about 90 passages alternately in young guineapig and rabbit to give a fixed virus having a shortened latent period of 3 to 4 days which was the shortest at that time [Jueki Chosasho Kenkyu Hokoku (Animal Plague Research Institute Study Report) No. 1, published on November, 1918].

Another typical rabies virus which has hitherto been known includes ERA strain, CVS strain, PV strain and the like. These rabies viruses have been confirmed to have a common structure constructed from five proteins i.e. L, G, N, M1 and M2. Among these virus-constituting proteins, it has been known that the G protein could be an effective material for preparing vaccine [Wiktor et al., J. Immunol. 110, 269 - 276 (1973)]. In recent years, monoclonal antibodies against each constituent protein of rabies virus have been prepared to enable comparison between rabies virus strains which had not yet been ascertained. Flamand et al. [J. Gen. Virol., 48, 105 - 109 (1980)] compared a number of rabies viruses on producibility of a neutralizing antibody and on the G protein associated with infection protection by employing monoclonal antibodies and showed that at least 14 antigenic determinants exist in the G protein and that the rabies virus strains can be distinguished from each other through comparison of these reaction patterns. Thereafter, many researchers have studied antigenicity of street viruses isolated in various areas in the world by employing a series of pannels of monoclonal antibody against the N protein. As a result, it has been found that, in many cases, the antigenic property of viruses varies depending on the area or the animal isolated therefrom. These facts may give a serious problem that a single vaccine strain might not be able to prevent rabies in all of the world. However, rabies vaccine of the present invention is derived from the Nishigahara strain, the superiority of which has already been proved by the fact that rabies has been eradicated in Japan as mentioned hereinabove.

Hitherto, there have already been reported some trials on the expression of rabies virus protein, especially G protein, by gene engineering technique. For example, L. T. Lawence et at. [cf. Vaccine 84, 203 - 208, Cold Spring Harbor Laboratory ed.] have reported that a gene coding for glycoprotein of rabies virus ERA strain was introduced into Escherichia coli expression system in order to express thereof, and as a result, although the glycoprotein gene was expressed in E. coli, a glycosylation did not occurred since the host cell was E. coli and hence an antigen effective as an immunogen was not obtained. Also E. Yelverton et al. [cf. Science, 219, 614 - 619 (1983)] have reported an experiment for expressing glycoprotein of rabies virus employing E. coli. This report does not show any result to have obtained an antigen effective as a vaccine, either. Thereafter, the expression of G protein was tried in an eucaryotic cell such as yeast or an animal cell as reported in Japanese Patent Publication (PCT) No. 500949/1986. Although this report describes the expression of glycosylated G protein, it does not include any immunological test result in animals.

As shown in the above-mentioned reports, although the expression of the G protein itself was successfully carried out, an effective rabies vaccine using these G proteins has not yet been developed in a practical point of view.

Under such circumstances, the present inventors have intensively studied on a development of rabies virus vaccine which is safer and more effective than the conventional rabies vaccine of the first generation by applying the genetic recombination technique. As a result, it has been found that the desired rabies glycoprotein can be obtained by the genetic recombination technique in accordance with the present invention, that is, by cloning cDNA coding for G protein of rabies virus derived from Nishigahara strain, inserting the cloned complementary DNA (cDNA) into an expression vector for eucaryotic cell in order to express in the eucaryotic cell. According to this process, the desired rabies virus glycoprotein is well expressed in the eucaryotic cell. It has also been confirmed that the expressed glycoprotein is effective as a vaccine. In accordance with the process for preparing rabies virus glycoprotein employing the genetic recombination technique of the present invention, the desired glycoprotein is expressed locally within cytoplasm without being coupled to a cell membrane of the transformed cell, and the expression of the desired glycoprotein can effectively be done with scarcely affecting a metabolism of the transformed cell, and further the protein can easily be purified. The thus obtained glycoprotein of the present invention not only reacts with a monoclonal antibody having an activity to neutralize rabies virus but also induces production of an antibody having an activity to neutralize rabies virus in animals when immunized therewith. Therefore, it was confirmed that the glycoprotein of the present invention is usefull for preparing vaccine or diagnostic reagent.

Fig. 1 shows a sequence of a structural gene coding for rabies G protein cloned by the present inventors and a gene sequence adjoining thereto as well as an amino acid sequence deducible therefrom.

Fig. 2 shows a removal of adenine cluster in the preparation of rabies G - cDNA.

Fig. 3 shows a construction of an expression vector pSVL-N913 employing COS cell as a host.

Fig. 4 shows a photograph displaying a morpholog ical change of COS cell wherein the rabies G protein expressed in the COS cell is shown by a fluorescent reaction.

Fig. 5 shows a construction of an expression vector pAMN913 for yeast.

The rabies virus strain employed in the present invention is derived from the Nishigahara strain as mentioned hereinbefore which has been kept through passage in rabbit for more than 2,000 passages till now (hereinafter referred to as "N●HL strain").

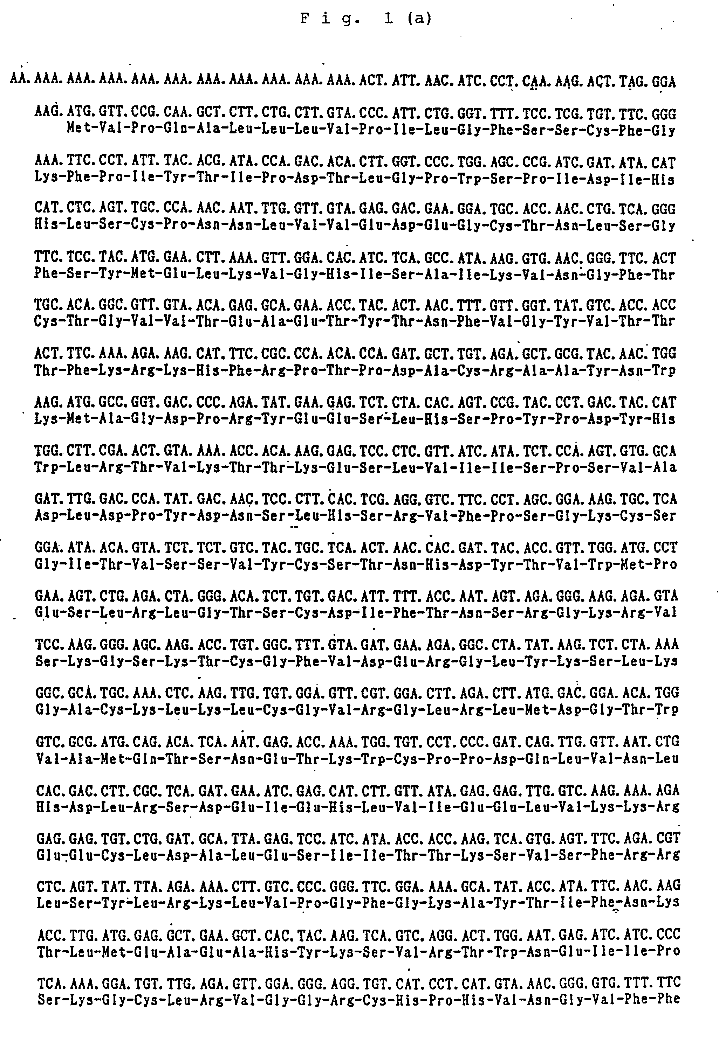

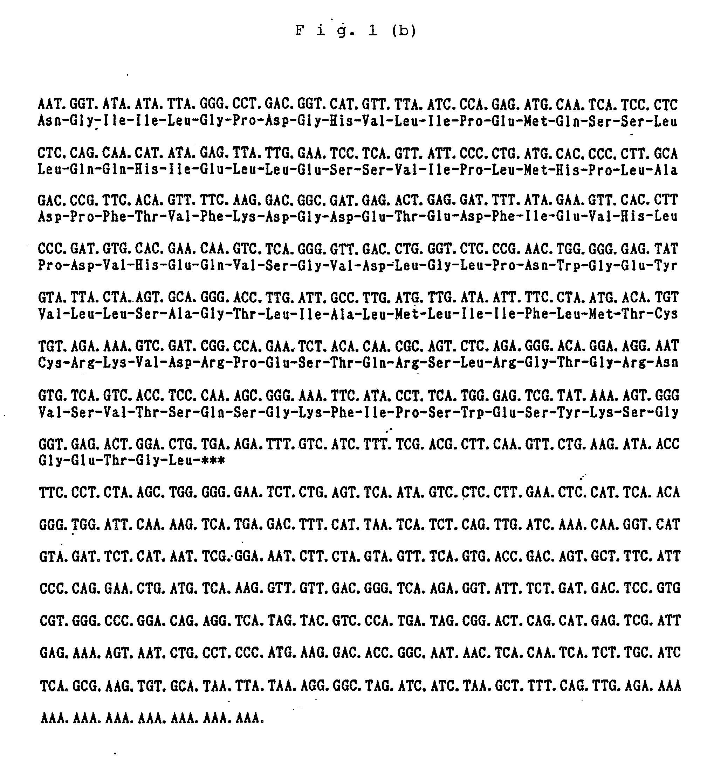

The gene fragment coding for rabies virus G protein of the present invention (G-cDNA) is a gene fragment containing 1575 base pairs starting from an initiation codon of ATG to a termination codon of TGA in the base pair shown in Fig. 1, or a gene fragment containing a gene sequence equivalent thereto, or a gene containing a portion of the gene which substantially codes for antigenic determinants in the above gene fragments.

The .G-cDNA of the present invention can be cloned from the rabies virus N●HL strain using a conventional gene cloning technique, for example, by a method of Gubler and Hoffman [cf. Gene 25, 263 - 269 (1983)]. E. coli containing a plasmid wherein the G-cDNA of the rabies virus N●HL strain is incorporated has been deposited by the present inventors as Escherichia coli DH5α/pUC-N913 at Fermentation Research Institute, Agency of Industrial Science and Technology, Japan under Budapest Treaty as "FERM BP-1613". The G-cDNA of the present invention can also be prepared by synthesizing the desired 1575 base pairs or a necessary part thereof by employing a DNA synthesizer (e.g. Type 381A manufactured by Applied Biosystem Co., Ltd.) based on the base pair shown in Fig. 1. The G-cDNA can be incorporated into a suitable vector and then introduced into an eucaryotic cell to express the desired rabies virus G protein in the cytoplasm.

For expressing the G-cDNA of the present invention, eucaryotic cells are employed as a host cell. Such eucaryotic cell includes yeast, an animal cell derived from a mammal or a culture cell derived from an insect cell. The expression system can be constructed by first constructing an expression vector suitable for a host cell employed and then introducing the expression vector into the host cell to give the desired transformed cell capable of expressing the G protein. Detailed explanation will be given in the following for both cases using yeast cell and culture cell as the host cell.

When the yeast cell is employed as the host cell, said G-cDNA is preferably incorporated into a shuttle vector which carries both yeast and E. coli genes and further a suitable promoter region. Such shuttle vector is a plasmid which contains a replication origin of yeast (e.g. 2 μori and/or arsl etc.), a selection marker gene in yeast, a promoter region derived from yeast, a replication origin of E. coli (e.g. ori derived from pBR322 etc.), and a selection marker gene in E. coli. The selection marker in yeast referred to herein means a gene for producing an amino acid such as leucine, histidine or tryptophan. The selection marker gene in E. coli means a gene resistant against ampicillin, kanamycin, tetracycline or chloramphenicol. The promoter region derived from yeast means an expression-control region capable of acting in the yeast cell. Such preferable promoter region includes, for example, a repressible acid phophatase (PH05) promoter, a glutaraldehyde-3-phosphate dehydrogenase (GAP-DH) promoter and the like. Preferable examples of such shuttle vector are pAM82 containing an expression-control region for acid phosphatase (promoter) (see U.S. Patent 4,778,761) and the like. Preferable yeast cell as the host includes, but not limited thereto, Saccharomyces cerevisiae AH22 (FERM BP-312) and the like.

For transforming yeast with the above-mentioned expression vector (shuttle vector) for yeast, the conventional transforming procedure [Ito et al., J. Bacteriol, 153, 163 - 168 (1983)] can be employed to give the transformed yeast cell capable of expressing the desired G protein.

When the culture cell is employed as the host, a part of a SV40 virus gene is replaced with the G-cDNA to give a recombinant virus capable of expressing the G protein in the transformed cell, the recombinant virus being then introduced into the host cell together with a helper virus to give the desired G protein. Alternately, a part of a gene of a virus capable of growing in the host cell in the state of a plasmid (e.g. papilloma virus etc.) is linked to the G-cDNA and the linked product is then introduced into the host cell to give a cell which can express the desired gene for the G protein in the state of a plasmid. Further, if E. coli plasmid, wherein the G-cDNA is incorporated and a virus or a promoter derived from a higher animal is linked to the upstream of said G-cDNA, is introduced into the culture cell, the G-cDNA incorporated into the host chromosome can be expressed in the transformed cell. For such plasmid, a commercially available one such as pSVL vector (manufactured by Pharmacia) can also be utilized.

The thus prepared transformant for expressing the G protein can be treated in the following manner so that the desired G protein is purified.

Firstly, the obtained transformant is cultured in a culture medium under conditions suitable for the employed cell so as to produce the desired G protein in cytoplasm of the transformant. When yeast cell is employed as the host cell, the collected cells are crushed by means of a physical treatment such as a ultrasonic treatment, treatment with glass beads or Manton Gaulin (APV-GAULIN, INC., USA, MS 18-8 TBS) optionally with a chemical treatment such as a treatment with a surfactant. In case of the culture cell employed as the host cell, a vigorous treatment as in the case of yeast cell is not necessary and only a treatment with a surfactant can elute the G protein produced in cytoplasm. The thus obtained lysate, after removing precipitates derived from the host cell by centrifuge and the like, is subjected to an affinity chromatography employing an anti-rabies G protein antibody to readily purify the desired G protein. Further purification can be conducted by a conventional purification procedure such as an ion exchange chromatography, a density gradient centrifuge, or a variety of affinity chromatographies, by which the protein can be highly purified to a degree of purity useful as a vaccine.

The rabies virus G-cDNA, the recombinant G protein prepared by the expression thereof and the expression system of the present invention are characteristic as compared to the conventional one in the following points:

(1) Base sequence of glycoprotein (G protein) cDNA:

Hitherto, cDNA base sequence has been reported on several rabies viruses wherein cDNA of mature G protein in the rabies virus constitutive protein comprises 1515 base pairs and codes for 505 amino acids. Comparison of the G-cDNA base sequence of the rabies virus N•HL strain of the present invention with those of the ERA strain, the CVS strain and the PV strain reveals that the G-cDNA of the present invention has a base sequence defferent from any of these strains, the difference being especially apparent in the portion of Asn-X-Thr(Ser) sequence where a glycosilation presumably occurs. The difference is summarized in Table 1.

Table 1

| Strain | Amino acid No. from N terminal | | | | | | |
|---|---|---|---|---|---|---|---|
| | 37 | 158 | 204 | 247 | 319 | 465 | 480 |
| N•HL | O | X | X | O | O | X | O |
| ERA | O | X | X | O | O | O | X |
| CVS | O | X | O | X | O | X | O |
| P V | O | O | X | O | O | O | X |
| O: the site being able to be glycosylated | | | | | | | |
| X: the site not being able to be glycosylated | | | | | | | |

A significance of the glycosylation is not clearly understood, but it is considered that it participates in the structure or stability of proteins. In fact, many proteins in living body such as physiologically active substances and serum proteins are glycoproteins. Although the presence of the glycosylation signal sequence does not necessarily mean the formation of glycosylation, there is a high possibility that this sequence participates in the structure or stability of proteins. In that sense, the glycosylation signal sequence participates in the function of proteins (i.e. biological activity), and hence, it is important to compare the glycosylation site as like as to compare the homology of whole cDNA.

(2) Expression type in animal culture cell system:

The expression plasmid of the present invention is introduced into an animal culture cell such as COS cell, and the rabies virus G protein-expressing site is detected by employing a polyclonal antibody which is prepared by immunization of an animal with the rabies virus G protein and a monoclonal antibody having an activity to neutralize the rabies virus.

As a result, the G protein is detected in cytoplasm as a granule but not at the cell membrane in accordance with the expression system of the present invention. Hithereto, such phenomenon in the expression of the rabies virus G protein in animal cells has never been reported and thus the above expression type in the present invention is quite novel. With respect to vesicular stomatitis virus (VSV) belonging to the same Rhabdoviridae as the rabies virus, Rose et al. have constructed a G protein cDNA of VSV in the same manner as in the expression system of the present invention and reported the expression type thereof [cf. Cell, 30, 753 - 762 (1982)]. According to this report, the specific antigen expressed in the transformed cell was observed to be in the state of being coupled to the cell membrane. This shows that the localization of the G protein in the present invention is based on a specific function of the G-cDNA of the present invention.

Except for the system where the expressed product is released into culture medium, the expression system for foreign gene may be classified into two types, i.e. the system where the expressed product is locally expressed within the cytoplasm and the system where the expressed product is expressed and localized in the state of being coupled to the cell membrane. Generally speaking, when the expressed product is coupled to the cell membrane, the host cell is susceptible of inhibition of growth, and further in a purification of protein (glycoprotein) associated with the cell membrane, a quality or yield of the desired protein is much affected since components of the cell membrane are coupled to the desired protein. The expression system for the rabies virus G protein according to the present invention can express the desired protein in the cytoplasm and thus is not accompanied with the above-mentioned problems of expression efficiency and purification, which enables to express and purify the desired protein much efficiently.

(3) Production of neutralizing antibody by the expressed G protein:

When a foreign gene is expressed by the genetic recombination technique using yeast cell or culture cell as in the present invention, the expressed peptide may occasionally not have properties suitable for biological preparation, even though it can react with an antibody to the desired peptide (antigen), i.e. it shows an antigenicity to the antibody. This may be depending on whether the peptide is modified so as to show a biological activity within the host cell after translation. As mentioned hereinbefore, E. Yelverton et al.

have successfully expressed the rabies G protein gene by employing E. coli and confirmed the reaction of the produced protein with an antibody against the rabies G protein, but reported that they could not produce any antibody when an animal is immunized with the produced protein. A major cause of this is presumed to be a lack of modification such as glycosylation in E. coli. Similarly in case that an eucaryotic cell such as yeast or a culture cell is employed, the same problem concerning the biological activity of the expressed product may arise in many cases. It is supposed that a variety of factors are related to this, including an effect of the expressed peptide against the host cell, a modification of the expressed peptide so as to show an inherent biological activity and the like. In other words, it is delicately affected depending on a variety of structural genes coding for the desired peptides which are introduced into the host cell for the expressiion thereof. On the contrary, when the G-cDNA of the present invention is introduced into the eucaryotic cell for the expression thereof, the produced G protein not only can react with an antibody to the G protein but also induce an antibody having an activity of neutralizing rabies virus when injected into an animal as a vaccine, which have been confirmed in an immunological test using guinea pig. Further, when the immunogenicity thereof was compared with that of purified G protein derived from rabies virus, both proteins showed the same level of immunogenicity. These show that the G protein of the present invention is sufficiently effective as a vaccine and suggest that a vaccine comprising the G protein of the present invention, which can be prepared at a low cost and in a large amount by means of the gene recombination technique, would contribute to extermination of rabies in developing countries and other surrounding countries.

The present invention is characterized by that the rabies virus G protein can be expressed in a localized state in the cytoplasm and not coupled to the cell membrane and that the thus obtained rabies G protein can induce a neutralizing antibody against rabies virus.

The present invention is illustrated by the following Examples, but should not be construed to be limited thereto.

In Examples, the following reagents and procedures were employed unless otherwise mentioned.

Enzyme and chemical substance

Restriction enzyme and modification enzyme employed are available from Takara Shuzo Co. Ltd., and TOYOBO Co. Ltd., respectively. For $\alpha$-$^{32}$P-dCTP, (Amasham code No. PB 10385, 800 Ci/mmol) was employed.

Cleavage, modification and ligation of DNA

Each enzyme was employed under conditions prescribed in a pamphlet "Takara Reagents for Genetic Engineering Research" and "TOYOBO Comprehensive Catalogue of Test for Genetic Engineering Research". After completion of the reaction of each enzyme, a phenol treatment and a chloroform treatment were conducted and DNA was collected by an ethanol precipitation in accordance with Molecular Cloning [T. Maniatis, p458 - 459 (1982)]. DNA ligation reaction was carried out by employing Takara Ligation Kit (Takara Shuzo Co. Ltd., Catalogue No. 6021) and in accordance with the attached protocol. Addition of a linker to the DNA terminal was also conducted by employing this kit and in accordance with the attached protocol.

Collection of DNA fragment by Agarose Electrophoresis

DNA agarose electrophoresis was conducted in accordance with Molecular Cloning [T. Maniatis, p150 - 163 (1982)]. Especially for extracting a particular DNA fragment from agarose gel, a low-melting agarose (BIO-RAD, Catalogue No. 162-0017) was employed and the collection thereof was also carried out in accordance with Molecular Cloning [T. Maniatis, p170 (1982)].

Extraction and purification of plasmid

When a large amount of plasmid is extracted and purified from transformed E. coli, the alkali method was employed as described in Molecular Cloning [T. Maniatis, p90 - 91 (1982)]. For selecting a colony transformed with the desired plasmid from a number of transformed E. coli, the desired positive clone was first selected in accordance with the following colony hybridization. According to Molecular Cloning [T. Maniatis, p368 (1982)], a small amount of plasmid was extracted and purified from that clone and then cleaved with a suitable restriction enzyme and subjected to an agarose electrophoresis for analysis.

Colony Hybridization and Plaque Hybridization

A probe was prepared from G-cDNA fragment by employing Nick Translation Reagent Kit (BRL Catalogue No. 8160SB) and Biotin-11-UTP (BRL Catalogue No. 9507SA) and according to a protocol attached to the kit. Colony hybridization and plaque hybridization were conducted under conditions according to Fujita et al. Saibo Kogaku (Cell Engineering) Vol. 4, No. 10. p893 - 900 (1985).

Example 1 (Preparation of G-cDNA)

(1) Extraction of RNA:

Hamster lung cells (HmLu cell), which had been grown at $37^\circ$ C for 48 hours, were infected with rabies virus N•HL strain at moi (multiplicity of infection) of 0.2. Forty eight (48) hours later, complete RNA was extracted from the cells in accordance with Molecular Cloning [T. Maniatis, p196 (1982)].

Rabies virus-infected cells of one roller bottle gave 1.5 mg of RNA measured by a spectroscopic quantitative determination (1.0 of adsorbance at 260 nm corresponds to 40 $\mu$g/ml). In order to confirm that the thus obtained RNA is not cleaved, 2 $\mu$g of the RNA was subjected to electrophoresis in accordance with P. Tomas [Pro. N.A.S. 77, 5201-5205 (1980)]. As a result, most of the RNA was 28s or 18s ribosome RNA and since these ribosome RNAs were not decomposed, it was estimated that G-mRNA contained therein in a small amount was not cleaved either.

The RNA subjected to electrophoresis according to P. Tomas was transfered to nitrocellulose (Schleicher & Schuell BA85) and a hybridization (Nothern blotting) was carried on the nitrocellulose using $^{32}$P-labelled rabies virus HEP-Flury strain G protein cDNA fragment as a probe. As a result, no band was observed for the RNA which was extracted from HmLu cells not infected with the virus and moved together in the electrophoresis, while bands were observed for the RNA extracted from virus-infected cells at around 2.2 kbp (kilo base pairs) and at a somewhat higher molecular site thereof. Hereinafter, this RNA extracted from virus-infected cells was employed for the synthesis of cDNA.

(2) Cloning of cDNA of rabies virus N•HL strain G protein:

The synthesis of cDNA was carried out in accordance with a method of Gubler and Hoffman [Gene, 25, 263 - 269 (1983)], i.e. a single stranded cDNA was synthesized by employing oligo $dT_{12-18}$ (manufactured by Pharmacia) as a primer, 5 $\mu$g of RNA extracted from virus-infected cells as a template, and a reverse transcription enzyme (Molony murine Leukemia Virus Transcriptase; manufactured by BRL). Then a nick was introduced into RNA chain hybridized with the thus synthesized single stranded cDNA with E. coli RNase H to prepare RNA chain and a double stranded cDNA was synthesized by employing the prepared RNA as a primer and E. coli polymerase I. The obtained double stranded cDNA was cloned by the following two methods.

[Procedure 1]

Employing Takara Ligation Kit, a double stranded cDNA wherein both ends were added with pSalI linker was reacted with a cloning vector pUC19, which was cleaved with the restriction enzyme SalI and then phosphate moiety was removed at the 5′ terminal thereof, in accordance with the protocol attached to the Takara Ligation Kit (at $16^\circ$ C for 30 minutes). The obtained product was introduced into a commercially available competent cell of DH5$\alpha$ (F$^-$ , endA1, hsdR17 (rk$^-$ , mk$^+$), supE44, thi-1, $\lambda$-recA1, gyr96, relA1, $\phi$80dlacZ$\Delta$M15). Then about 3,000 clones thus obtained were subjected to a colony hybridization employing rabies virus HEP-Flury strain G protein cDNA fragment as a probe. As a result, a color was developed in one clone, which was designated as "pUC-RNSL". This cDNA was treated in the same manner as described in "construction of G protein expression system" and then introduced into COS cell and yeast expression systems.

[Procedure 2]

According to Nagata et al., Jikken Igaku (Experimental Medicine), Vol. 4, No. 8, 87 - 92 (1986), the double stranded cDNA (0.5 μg) was firstly treated with EcoRImethylase to methylate the EcoRI cleaved sites and pEcoRI linkers were added to both ends of the DNA. For cloning the DNA, a ligation was carried out between this DNA and λgt10 of an arm cleaved with EcoRI (available from STRATAGENE) (GT10) in accordance with a protocol described in the pamphlet. Then G-cDNA was cloned by employing the in vitro packaging procedure using an in vitro packaging extract available from STRATAGENE (trade name: Giga pack puls, manufactured by STRATAGENE) and the host cell of VCS258 attached thereto or C600hfl. About 350,000 clones were obtained in total. Seven thousand (7,000) clones were placed on a single plate and the plaque hybridization was carried out to give 21 positive clones.

(3) Determination of whole base sequence of pRNSL:

In order to prepare a G-cDNA fragment, pRNSL (20 μg) was digested with the restriction enzyme SalI (50 Units) employing buffer attached thereto at 37°C for 4 hours. The obtained DNAs were separated into two fragments by means of a 1 % low-melting agarose electrophoresis and a G-cDNA fragment corresponding to 2.1 kbp was extracted and recovered.

(a) For further fragmentation of this G-cDNA fragment, the G-cDNA fragment (0.1 μg each) was cleaved with each four base-recognizing restriction enzyme HaeIII, RsaI, AluI, TaqI or Sau3AI into 6, 5, 6, 9 or 7 fragment, respectively. Among these fragmented G-cDNAs, the fragment cleaved with HaeIII, RsaI or AluI was ligated with M13mp18 cleaved with SmaI or M13mp19 cleaved with both SmaI and SalI, the TaqI fragment was ligated with M13mp18 cleaved with AccI, and the Sau3AI fragment was ligated with M13mp19 cleaved with BamHI or cleaved with both BamHI and SalI, by employing the Takara ligation kit.

pUC-RNSL was cleaved with restriction enzymes KpnI and BamHI. The deletion was conducted for the cleaved pUC-RNSL at from the BamHI cleaved site by employing a Takara Kilosequence Deletion Kit (Catalogue No. 6030) and a protocol attached thereto to prepare G-cDNAs with a different deletion degree, which was cleaved with SalI. The thus obtained deleted G-cDNA was ligated with M13mp18 cleaved with both SmaI and SalI by means of the Takara Ligation Kit.

In accordance with the procedure of Toyobo Instruction Manual, a competent cell of JM101 was transformed with the DNA treated in the above procedures (a) and (b). The procedure of the transformation and following extraction and purification of a single stranded DNA were conducted in accordance with Toyobo M13 Cloning Kit (Code No. M13-001) and "instruction manual" thereof. The sequencing of the thus obtained single stranded DNA was carried out in accordance with BRL sequencing gel electrophoresis system employing Toyobo M13 Sequencing Kit (Code No. M13-003) and BRL electrophoresis apparatus (Model S2 Catalogue No. 1105) and a protocol attached thereto. As a result, it was found that the pRNSL comprised 2,123 base pairs (bp) which contained an open reading frame of 1,575 bp coding for rabies G protein comprising 524 amino acids (see Fig. 1).

As a result of the sequencing, the cloned G-cDNA was found to contain a cluster of 32 adenines at the 5' end. It was expected that, when the cloned G-cDNA was linked to the downstream of the SV40 late region promoter or yeast acid phosphatase promoter, the adenine cluster would greatly inhibit the transcription thereof. Therefore, the adenine cluster was removed in the following manner. Firstly, pUC-RNSL (2 μg) was cleaved with the restriction enzyme PstI at 37°C for 2 hours. The obtained DNA was digested by means of Takara nuclease Ba131-S (Catalogue No. 2510A) at 30°C for 2 minutes employing the following reaction compositions:

| DNA (cleaved with PstI) | 23 μl |
| 5xBa131 buffer (*) | 6 μl |
| Ba131S (2 Units/ml) | 1 μl |

(*) 5xBa131 buffer: 100 mM Tris-HCl, 3M NaCl, 60 mM CaCl₂, 60 mM MgCl₂, 5 mM EDTA

Thereafter, pSall linkers (Toyobo SAL-801, 2 μg) was added to both ends of the above nuclease Ba131 digested DNA by employing the Takara Ligation Kit. The DNA was sufficiently cleaved with the restriction enzyme Sall and subjected to low-melting agarose electrophoresis. A broad DNA band at around 2.1 kbp was cut off and the DNA was collected. The collected DNA was cloned by ligating the DNA with pUC19 cleaved with the restriction enzyme Sall with the Ligation Kit and transforming a competent cell DH5α - (available from BRL, Catalogue No. 8263SA) with the ligate.

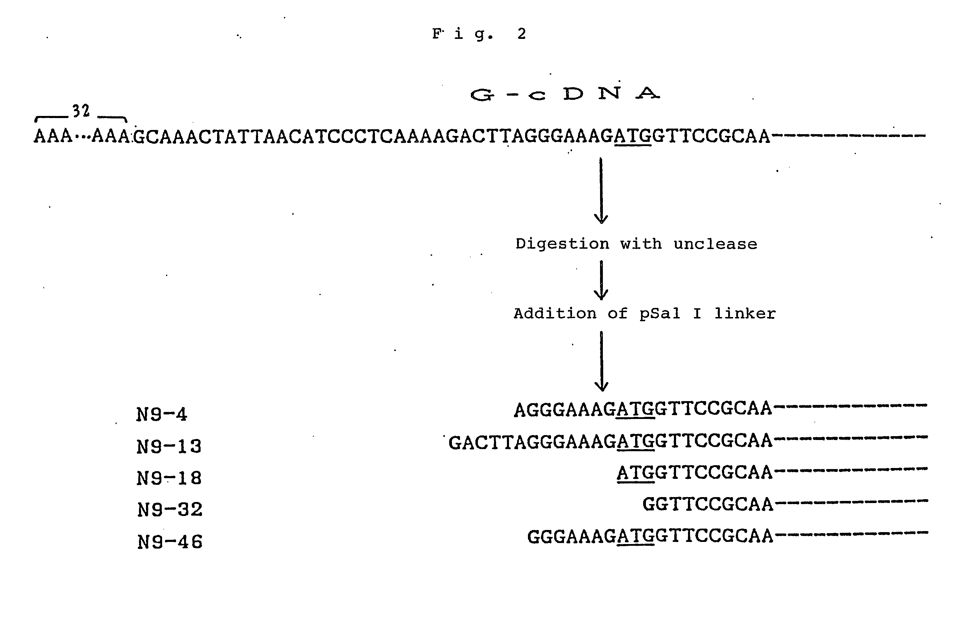

The base sequence of the thus obtained clone was determined at the 5' end thereof in accordance with the Toyobo M13 Cloning Kit (Code No. M13-001) and "instruction manual" thereof (see Fig. 2). Among them, N9-13 was employed as the G protein cDNA for introducing into the expression systems of COS cell and yeast in the following procedure. The plasmid containing this gene fragment has been deposited in the state of being incorporated into E. coli as Escherichia coli DH5α/pUC-N913 at Fermentation Research Institute, Agency of Industrial Science and Technology, Japan under Budapest Treaty as "FERM BP-1631".

Example 2 (Expression of glycoprotein in an animal cell as a host)

(1) Construction of COS cell expression plasmid:

In order to isolate G protein cDNA inserted in pUC-N913, pUC-N913 was cleaved with the restriction enzyme Sall to separate into two fragments. In accordance with Molecular Cloning [T. Maniatis, 170 (1982)], a G-cDNA fragment was obtained by means of a low-melting agarose gel electrophoresis.

pSVL, commercially available from Pharmacia (Catalogue No. 27-4509-01), was employed for the expression vector. In order to incorporate G-cDNA into the downstream of the promoter of the expression vector, the vector was cleaved with the restriction enzyme XhoI and the phosphate at the 5' end thereof was removed. Then pSVL was ligated with N9-13G-cDNA by means of the Takara ligation kit and a HB101 competent cell was infected with this ligate.

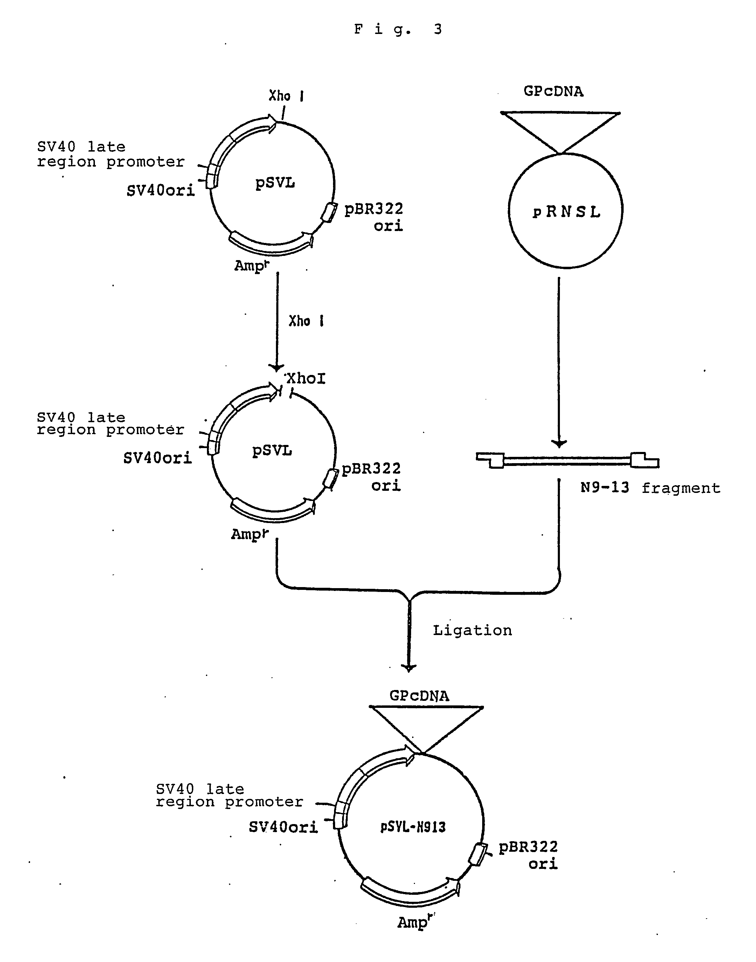

Employing the procedure described in the above "Extraction and purification of plasmid" and "Colony hybridization and plaque hybridization", the ligate wherein N9-13G-cDNA was incorporated in the transcription direction of SV40 late region promoter was selected and a COS cell was infected with one of such ligate PSVL-N913 (Fig.3) by means of Cell Phect Transfection Kit (Catalogue No. 17-0595-01) which is available from Pharmacia.

(2) Confirmation of expression of G protein in COS cell:

SV40-transformed cell derived from Africa green monkey kidney (COS-1, available from Dainippon Pharmaceutical Co. Ltd., Catalogue No. 09-1650) was cultured in CO₂ incubator at 37°C for 24 hours so that $10^5$ cells/ml of cell concentration is obtained in MEM medium containing 10 % fetal bovine serum. After culturing for 24 hours, the medium was removed and the cells were once washed with 0.01 M PBS. A mixture of DEAE-dextrane solution (50 μg/ml, 100 μl) and G protein expression vector pSVL-N913 (0.5 μg) was added dropwise to the cell, which was allowed to stand at room temperature for 15 minutes to infect the cell. Thereafter, the cell was washed with MEM medium without serum and was cultured under usual conditions (37°C, 5 % CO₂ incubator) for 48 hours.

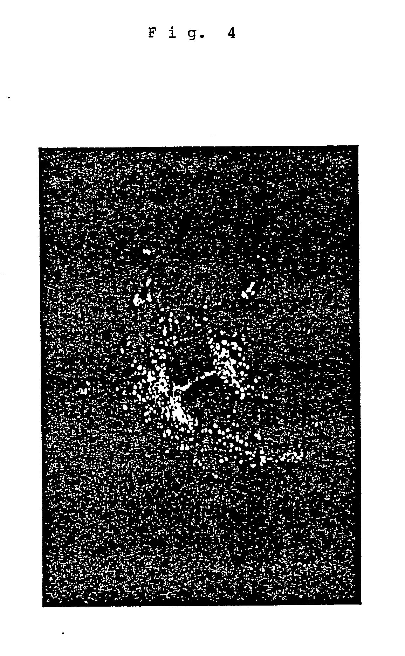

Then, in order to confirm that the thus obtained cells have been expressed the G protein, the cells were observed by the indirect fluorescent antibody technique in accordance with the procedure described in "Uirusugaku Jikkenho (Virological Experiment)" (National Institute of Health, alumni association ed., p297 - 329). That is, 48 hours after the DNA infection, culture supernatent was removed and a pretreatment was conducted by dropwise addition of acetone to prevent exfoliation or elution of the desired antigen. The pretreated cells were reacted with a monoclonal antibody having a neutralizing activity as a primary antibody at room temperature for 10 minutes. After washing with 0.01 M PBS, the cells were reacted with anti-mouse IgG labelled with FITC (fluorescen isothiocyanate) of a fluorescent pigment (FITC-Rbx Mouse IgG(H + L), manufactured by ZYMED, Catalogue No. 61-6511). The reacted cells were washed with 0.01 M PBS and observed with a fluorescence microscope. Fig. 4 shows the thus observed fluorescence reaction of the COS cells. As a result, it was confirmed that the G protein expressed in the COS cell was expressed in cytoplasm as a granule not in the state of being coupled to the cell membrane.

Example 3 (Expression of glycoprotein in yeast cells as a host)

(1) Construction of yeast expression plasmid:

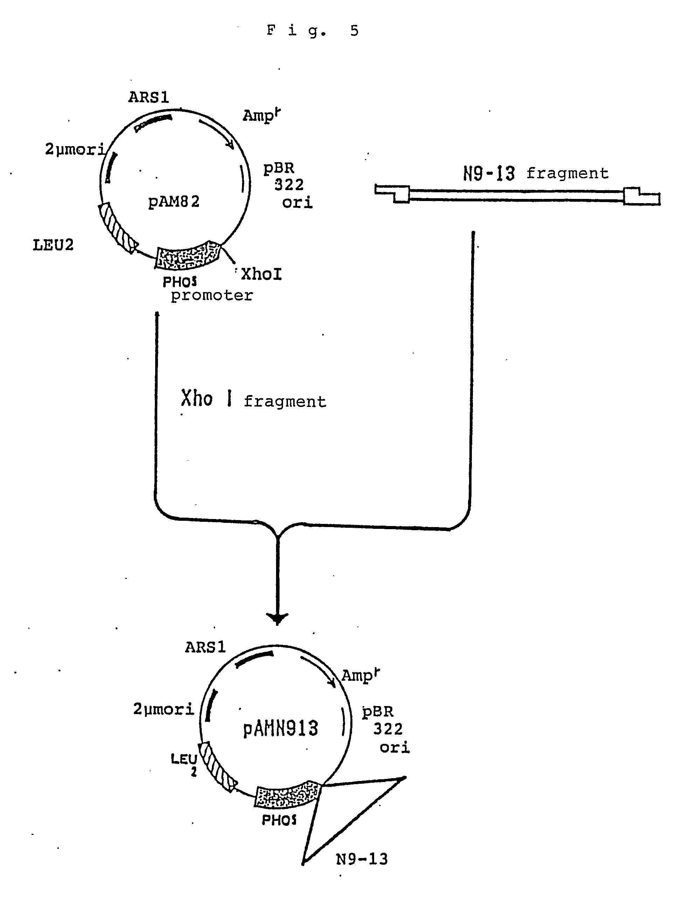

In order to isolate G protein cDNA inserted in pUCN913, pUC-N913 was cleaved with the restriction enzyme SalI to separate into two fragments. A G-cDNA fragment was obtained by means of a low-melting agarose gel electrophoresis. On the other hand, an expression vector in yeast pAM82 (see U.S. Patent 4,778,761) was cleaved with the restriction enzyme XhoI. Thereafter, the phosphate at the 5′ end was removed with alkaline phophatase (manufactured by Boeringer, articles No. 703023) in accordance with Molecular cloning (T. Maniatis, p133 - 134). Then this vector was ligated with N9-13 by means of the Takara ligation kit and a HB101 competent cell of E. coli was infected with this ligate. The infected E. coli was subjected to a colony hybridization employing G-cDNA as a probe to give a positive clone pAMN913 (see Fig.5).

(2) Expression of glycoprotein in yeast cell:

Saccharomyces cerevisiae AH22 [a leu2 his4 Can1 (Cir+)] (FERM BP-312) of yeast was inoculated on YPE medium (2 % polypeptone, 1 % yeast extract, 2 % glucose) (100 ml) and cultured at 30° C overnight, and thereafter, the cells were collected by centrifugation. The cells thus collected were washed with sterilized water (20 ml), suspended in a solution (5 ml) of 1.2 M sorbitol and Zymolyase-60,000 (manufactured by Seikagaku Kogyo K.K., Japan, 100 μg/ml), and the suspension was allowed to stand at 30° C for 30 minutes to give spheroplast. The spheroplast thus prepared was washed with a 1.2 M sorbitol solution three times, and then suspended in a solution (0.6 ml) of 2 M sorbitol, 10 mM $CaCl_2$ and 10 mM Tris-HCl (pH 7.5). The suspension thus prepared was divided into a small test tube in a volume of 60 μl. To the suspension was added the solution of the recombinant plasmid pAMN913 (10μg) prepared as above. After well mixing, 0.1 M $CaCl_2$ (3μg) was added thereto in a final concentration of 10 mM $CaCl_2$, and the mixture was allowed to stand at room temperature for 5 to 10 minutes. To the resulting mixture was added each 1 ml of a solution of 20 % polyethylene glycol 4,000, 10 mM $CaCl_2$ and 10 mM Tris-HCl (pH 7.5), and the mixture was allowed to stand at room temperature for 20 minutes. The resulting mixture (each 0.2 ml) was added to a medium (10 ml) consisting of 22 % sorbitol, 2 % glucose, 0.7 % yeast nitrogen base amino acid. 2 % YPD, 20 μg/ml histidine and 3 % agar, which was kept at a constant temperature of 45° C. After gently mixing, the mixture was added in a layer onto a plate of minimal medium containing 1.2 M sorbitol which has previously been prepared and consisted of 0.7 % yeast nitrogen base amino acid, 2 % glucose, 20 μg/ml histidine and 2 % agar and was set thereon. The plate was incubated at 30° C to give a colony of a leucine-non-requiring yeast. The colony was incubated in a BurkHolder minimal medium supplemented with histidine (20 μg/ml) [see Tohe, A. et al; J. Bacterol., 113, 117 - 738 (1973)] to give the desired transformed yeast Saccharomyces cerevisiae.

The colony of the transformed yeast thus obtained was applied onto an agar place of BurkHolder minimal medium supplemented with histidine (20 μg/ml) and incubated at 30° C to form a colony (in order to confirm the transformant requiring no leucine). The resulting cells were separated from the colony, inoculated in BurkHolder minimal medium (10 ml) supplemented with histidine (2u μg/ml) and incubated at 30° C. After about 24 hours, the cells in logarithmic growth phase were collected by centrifugation and were suspended in a minimal medium (10 ml) containing no phosphoric acid (which was prepared by replacing $KH_2PO_4$ in BurkHolder minimal medium with KCl, followed by supplementing 20 μg/ml histidine) in a cell concentration of about $4 \times 10^6$ cells/ml. After incubating at 30° C for 24 hours, the culture broth was centrifuged at 4,000 r.p.m. for 10 minutes to collect the cells.

The cells thus separated were suspended in a solution (3 ml) of 1.2 M sorbitol, 50 mM phophate buffer (pH 7.2), 14 mM 2-mercaptoethanol and 100 μg/ml Zymolyase-60,000 (manufactured by Seikagaku Kogyo K.K. Japan), and the mixture was gently shaken at 30°C for 30 minutes to give spheroplast. The spheroplast was collected by centrifugation and was well suspended in 50 mM phophate buffer (pH 7.2, 1 ml) containing 1 % Triton X-100, and the mixture was vigorously stirred with glass beads to fracture the cells.

The resulting fractured mixture was centrifuged at 5,000 r.p.m. for 10 minutes, and the resulting supernatant was taken as the yeast-lysed solution. As to the supernatant, the G protein antigen activity was measured by the following procedure, i.e. an-anti-G protein monoclonal antibody as the first antibody was diluted with 50 mM carbonate buffer (pH 9.5), added to a 96-well plate for ELISA and incubated at 37°C for 2 hours or at room temperature for 4 hours to coat the monoclonal antibody on the plate. The coated plate was washed with PBS (phosphate buffered saline) / 0.05 % Tween 20 solution (PBS-Tween) three times and masked with PBS-Tween containing 1 % BSA (BSA-PBS-Tween) at 4°C overnight. Then, each sample was suitably diluted with PBS-Tween, added to each well and kept at 37°C for 1 hour. Then the plate was washed with PBS-Tween five times and a BSA-PBS-Tween solution containing an anti-G protein monoclonal antibody labelled with peroxidase was added to the plate and kept at 37°C for 1 hour. The plate was again washed with PBS-Tween five times and an EDTA solution of TMBZ (N,N'-tetramethylbenzidine hydrochloride) (0.006 % hydrogen peroxide) as a substrate was added to the plate for development. A degree of the development was measured with an autoreader (OD 450 nm) to give the results shown in Table 2. As a negative control, the G protein antigen activity was also measured for yeast which was transformed with the plasmid pAM82 wherein the G-cDNA was not incorporated and treated in the same manner as above.

Table 2

|  | pAMN913 | pAM82 |
|---|---|---|
| OD 450 | 1.7 | 0.1 |

As is clear from the result shown in Table 2, a very high G protein antigen activity was detected in only yeast which was incorporated with the shuttle vector wherein the G-cDNA of the present invention was inserted.

Example 4 (Immunogenicity test in guinea pig)

(1) Preparation of immunization antigen and immunization:

Employing an affinity chromatography in which a monoclonal antibody having a virus-neutralizing activity was used as a ligand, a G protein for use in immunization test was purified from a yeast lysate transformed with pAMN913. To the purified G protein was added aluminum hydroxide gel to prepare an immunization antigen containing 1 μg of the G protein per dose. Twenty (20) Hartley guinea pigs (female) weighing 450 to 650 g were used. The immunization antigen prepared as above was intraperitoneally injected to ten (10) guinea pigs and one week after the injection, a booster was injected. The remaining ten (10) guinea pigs were non-immunized control group where a physiological saline was injected in the same manner.

(2) Neutralization test:

As a neutralization virus, there was employed rabies virus N•HL strain which was acclimatized in the HmLu-1 cell and had a toxicity capable of leading matured mice to death by injection thereof into the brain. Blood was taken before the first injection and after the booster injection from all animals of immunization group. Blood was also taken from the animals of non-immunization group in the same manner. Then these sera were heated at 56°C for 30 minutes for inactivation. The inactivated serums were diluted twofold and 50 μl thereof were reacted with a virus solution (50 μl) which contained virus in an amount of 200 $TCID_{50}$ - (50 % Tissue Culture Infective Dose) at 37°C for 90 minutes. Each 50 μl of the reaction mixture was then incubated in a 96-well microplate. The incubated reaction mixture was inoculated in HmLu-1 cells and absorbed at 37°C for 1.0 hour, followed by addition of a cell-maintaining medium (Eagle MEM medium) (150 μl) to culture at 37°C for 4 days. After the culture, the plate was reacted with an anti-rabies

11

glycoprotein monoclonal antibody labelled with horseradish peroxidase (labelled antibody). When the serum did not react with the labelled antibody it means that the serum was not infected with virus and it was positive in the neutralizing antibody. A neutralizing antibody titer was shown by a reciprocal number of a maximal dilution of the serum which completely inhibited the virus infection. As a result, it was found that the serum obtained from immunization with the G protein of the present invention had the neutralizing antibody. The obtained results are shown in Table 3.

## Table 3

| | Immuniz. group | | | Non-immuniz. group | | |
|---|---|---|---|---|---|---|
| Guinea pig No. | Neutraliz. Ab titer | | Guinea pig No. | Neutraliz. Ab titer | | |
| | Before inj. (0w) | After inj. (3w) | | Before inj. (0w) | After inj. (3w) | |
| 1 | < 2 | 32 | 11 | < 2 | < 2 | |
| 2 | < 2 | 8 | 12 | < 2 | < 2 | |
| 3 | < 2 | 16 | 13 | < 2 | < 2 | |
| 4 | < 2 | 64 | 14 | < 2 | < 2 | |
| 5 | < 2 | 32 | 15 | < 2 | < 2 | |
| 6 | < 2 | 4 | 16 | < 2 | < 2 | |
| 7 | < 2 | 32 | 17 | < 2 | < 2 | |
| 8 | < 2 | 64 | 18 | < 2 | < 2 | |
| 9 | < 2 | 16 | 19 | < 2 | < 2 | |
| 10 | < 2 | 128 | 20 | < 2 | < 2 | |

(Note) 0w: at 0 week    3w: at the third week

(4) Virus challenge test:

Guinea pigs were booster-injected in accordance with the procedure described in the above item (2) and inoculated (challenged) with Rabies virus CVS strain (about 10 $LD_{50}/0.2$ ml) within masticatory muscle two weeks after the booster injection (at the third week from the first injection). The same challenge was also conducted for the control group. Occurrence of symptoms were observed for two weeks after the challenge. Guinea pigs having no symptoms during two weeks were referred to as positive for protection. The results are shown in Table 4.

Table 4

| Test group | Death No. | No. showed no symptoms | protection rate (%) |
|---|---|---|---|
| Immuniz. group | 1 | 9 | 90 |
| Control group | 10 | 0 | 0 |

As clearly shown in the above Table 4, all guinea pigs except for one (No. 6 in Table 3) showed no symptoms in the group wherein the animals were immunized with the G protein of the present invention, while all guinea pigs in the non-immunizing control group died with typical symptoms of rabies. From these experimental results, it is confirmed that the G protein of the present invention is extremely useful as a rabies vaccine.

**Claims**

1. A gene fragment containing at least a part of the following base sequence coding for a rabies virus glycoprotein or of a base sequence equivalent thereto:

```
        10         20         30         40         50
ATGGTTCCGC AAGCTCTTCT GCTTGTACCC ATTCTGGGTT TTTCCTCGTG

        60         70         80         90        100
TTTCGGGAAA TTCCCTATTT ACACGATACC AGACACACTT GGTCCCTGGA

       110        120        130        140        150
GCCCGATCGA TATACATCAT CTCAGTTGCC CAAACAATTT GGTTGTAGAG

       160        170        180        190        200
GACGAAGGAT GCACCAACCT GTCAGGGTTC TCCTACATGG AACTTAAAGT

       210        220        230        240        250
TGGACACATC TCAGCCATAA AGGTGAACGG GTTCACTTGC ACAGGCGTTG

       260        270        280        290        300
TAACAGAGGC AGAAACCTAC ACTAACTTTG TTGGTTATGT CACCACCACT

       310        320        330        340        350
TTCAAAAGAA AGCATTTCCG CCCAACACCA GATGCTTGTA GAGCTGCGTA

       360        370        380        390        400
CAACTGGAAG ATGGCCGGTG ACCCCAGATA TGAAGAGTCT CTACACAGTC

       410        420        430        440        450
CGTACCCTGA CTACCATTGG CTTCGAACTG TAAAAACCAC AAAGGAGTCC

       460        470        480        490        500
CTCGTTATCA TATCTCCAAG TGTGGCAGAT TTGGACCCAT ATGACAACTC

       510        520        530        540        550
CCTTCACTCG AGGGTCTTCC CTAGCGGAAA GTGCTCAGGA ATAACAGTAT

       560        570        580        590        600
CTTCTGTCTA CTGCTCAACT AACCACGATT ACACCGTTTG GATGCCTGAA

       610        620        630        640        650
AGTCTGAGAC TAGGGACATC TTGTGACATT TTTACCAATA GTAGAGGGAA

       660        670        680        690        700
GAGAGTATCC AAGGGGAGCA AGACCTGTGG CTTTGTAGAT GAAAGAGGCC

       710        720        730        740        750
TATATAAGTC TCTAAAAGGC GCATGCAAAC TCAAGTTGTG TGGAGTTCGT
```

13

```
        760        770        780        790        800
GGACTTAGAC TTATGGACGG AACATGGGTC GCGATGCAGA CATCAAATGA

        810        820        830        840        850
GACCAAATGG TGTCCTCCCG ATCAGTTGGT TAATCTGCAC GACCTTCGCT

        860        870        880        890        900
CAGATGAAAT CGAGCATCTT GTTATAGAGG AGTTGGTCAA GAAAAGAGAG

        910        920        930        940        950
GAGTGTCTGG ATGCATTAGA GTCCATCATA ACCACCAAGT CAGTGAGTTT

        960        970        890        990       1000
CAGACGTCTC AGTTATTTAA GAAAACTTGT CCCCGGGTTC GGAAAAGCAT

       1010       1020       1030       1040       1050
ATACCATATT CAACAAGACC TTGATGGAGG CTGAAGCTCA CTACAAGTCA

       1060       1070       1080       1090       1100
GTCAGGACTT GGAATGAGAT CATCCCCTCA AAAGGATGTT TGAGAGTTGG

       1110       1120       1130       1140       1150
AGGGAGGTGT CATCCTCATG TAAACGGGGT GTTTTTCAAT GGTATAATAT

       1160       1170       1180       1190       1200
TAGGGCCTGA CGGTCATGTT TTAATCCCAG AGATGCAATC ATCCCTCCTC

       1210       1220       1230       1240       1250
CAGCAACATA TAGAGTTATT GGAATCCTCA GTTATTCCCC TGATGCACCC

       1260       1270       1280       1290       1300
CCTTGCAGAC CCGTTCACAG TTTTCAAGGA CGGCGATGAG ACTGAGGATT

       1310       1320       1330       1340       1350
TTATAGAAGT TCACCTTCCC GATGTGCACG AACAAGTCTC AGGGGTTGAC

       1360       1370       1380       1390       1400
CTGGGTCTCC CGAACTGGGG GGAGTATGTA TTACTAAGTG CAGGGACCTT

       1410       1420       1430       1440       1450
GATTGCCTTG ATGTTGATAA TTTTCCTAAT GACATGTTGT AGAAAAGTCG

       1460       1470       1480       1490       1500
ATCGGCCAGA ATCTACACAA CGCAGTCTCA GAGGGACAGG AAGGAATGTG

       1510       1520       1530       1540       1550
TCAGTCACCT CCCAAAGCGG GAAATTCATA CCTTCATGGG AGTCGTATAA

       1560       1570
AAGTGGGGGT GAGACTGGAC TGTGA
```

wherein the equivalent base sequence is related to the above base sequence in that it encodes the same amino acid sequence or in that it hybridizes to the above base sequence under conventional conditions.

2. The gene fragment according to claim 1 which contains the base sequence from position 58 (adenine) to position 1575 (adenine) of the base sequence coding for the rabies virus glycoprotein according to claim 1.

3. A recombinant rabies virus glycoprotein prepared by expressing the gene fragment according to claim 1 in eukaryotic cells.

4. A process for preparing a rabies virus glycoprotein which comprises introducing into eukaryotic cells an expression vector wherein the gene fragment according to claim 1 is inserted downstream of a promoter capable of functioning in eukaryotic cells, and culturing said transformed cells to yield a rabies virus glycoprotein in the cytoplasm of said cells.

5. The process according to claim 4, wherein the eukaryotic cells are yeast cells.

6. The process according to claim 4, wherein the expression vector is a shuttle vector being a plasmid containing an origin of replication of yeast, a selectable marker gene for yeast, a promoter region derived from yeast, an origin of replication of Escherichia coli, and a selectable marker gene for E. coli.

7. A process for preparing a rabies virus glycoprotein which comprises introducing into cultured cells a recombinant virus into which a gene fragment is inserted encoding a rabies virus G protein, and culturing said transformed cells to yield a rabies virus glycoprotein in the cytoplasm of said cells.

8. The process according to claim 7, wherein the recombinant plasmid is prepared by replacing a part of an SV40 virus gene with a gene fragment coding for a rabies virus G protein, and wherein the cultured cells are transfected with said recombinant plasmid.

9. The process according to claim 7, wherein the recombinant virus is prepared by linking a part of a virus capable of growing in the cultured cells in the state of a plasmid to a gene fragment coding for a rabies virus G protein.

10. Escherichia coli DH5α/pUC-N913 (FERM BP-1631).

F i g. 1 (a)

```
AA. AAA. AAA. AAA. AAA. AAA. AAA. AAA. AAA. AAA. AAA. ACT. ATT. AAC. ATC. CCT. CAA. AAG. ACT. TAG. GGA


AAG. ATG. GTT. CCG. CAA. GCT. CTT. CTG. CTT. GTA. CCC. ATT. CTG. GGT. TTT. TCC. TCG. TGT. TTC. GGG
     Met-Val-Pro-Gln-Ala-Leu-Leu-Leu-Val-Pro-Ile-Leu-Gly-Phe-Ser-Ser-Cys-Phe-Gly


AAA. TTC. CCT. ATT. TAC. ACG. ATA. CCA. GAC. ACA. CTT. GGT. CCC. TGG. AGC. CCG. ATC. GAT. ATA. CAT
Lys-Phe-Pro-Ile-Tyr-Thr-Ile-Pro-Asp-Thr-Leu-Gly-Pro-Trp-Ser-Pro-Ile-Asp-Ile-His


CAT. CTC. AGT. TGC. CCA. AAC. AAT. TTG. GTT. GTA. GAG. GAC. GAA. GGA. TGC. ACC. AAC. CTG. TCA. GGG
His-Leu-Ser-Cys-Pro-Asn-Asn-Leu-Val-Val-Glu-Asp-Glu-Gly-Cys-Thr-Asn-Leu-Ser-Gly


TTC. TCC. TAC. ATG. GAA. CTT. AAA. GTT. GGA. CAC. ATC. TCA. GCC. ATA. AAG. GTG. AAC. GGG. TTC. ACT
Phe-Ser-Tyr-Met-Glu-Leu-Lys-Val-Gly-His-Ile-Ser-Ala-Ile-Lys-Val-Asn-Gly-Phe-Thr


TGC. ACA. GGC. GTT. GTA. ACA. GAG. GCA. GAA. ACC. TAC. ACT. AAC. TTT. GTT. GGT. TAT. GTC. ACC. ACC
Cys-Thr-Gly-Val-Val-Thr-Glu-Ala-Glu-Thr-Tyr-Thr-Asn-Phe-Val-Gly-Tyr-Val-Thr-Thr


ACT. TTC. AAA. AGA. AAG. CAT. TTC. CGC. CCA. ACA. CCA. GAT. GCT. TGT. AGA. GCT. GCG. TAC. AAC. TGG
Thr-Phe-Lys-Arg-Lys-His-Phe-Arg-Pro-Thr-Pro-Asp-Ala-Cys-Arg-Ala-Ala-Tyr-Asn-Trp


AAG. ATG. GCC. GGT. GAC. CCC. AGA. TAT. GAA. GAG. TCT. CTA. CAC. AGT. CCG. TAC. CCT. GAC. TAC. CAT
Lys-Met-Ala-Gly-Asp-Pro-Arg-Tyr-Glu-Glu-Ser-Leu-His-Ser-Pro-Tyr-Pro-Asp-Tyr-His


TGG. CTT. CGA. ACT. GTA. AAA. ACC. ACA. AAG. GAG. TCC. CTC. GTT. ATC. ATA. TCT. CCA. AGT. GTG. GCA
Trp-Leu-Arg-Thr-Val-Lys-Thr-Thr-Lys-Glu-Ser-Leu-Val-Ile-Ile-Ser-Pro-Ser-Val-Ala


GAT. TTG. GAC. CCA. TAT. GAC. AAC. TCC. CTT. CAC. TCG. AGG. GTC. TTC. CCT. AGC. GGA. AAG. TGC. TCA
Asp-Leu-Asp-Pro-Tyr-Asp-Asn-Ser-Leu-His-Ser-Arg-Val-Phe-Pro-Ser-Gly-Lys-Cys-Ser


GGA. ATA. ACA. GTA. TCT. TCT. GTC. TAC. TGC. TCA. ACT. AAC. CAC. GAT. TAC. ACC. GTT. TGG. ATG. CCT
Gly-Ile-Thr-Val-Ser-Ser-Val-Tyr-Cys-Ser-Thr-Asn-His-Asp-Tyr-Thr-Val-Trp-Met-Pro


GAA. AGT. CTG. AGA. CTA. GGG. ACA. TCT. TGT. GAC. ATT. TTT. ACC. AAT. AGT. AGA. GGG. AAG. AGA. GTA
Glu-Ser-Leu-Arg-Leu-Gly-Thr-Ser-Cys-Asp-Ile-Phe-Thr-Asn-Ser-Arg-Gly-Lys-Arg-Val


TCC. AAG. GGG. AGC. AAG. ACC. TGT. GGC. TTT. GTA. GAT. GAA. AGA. GGC. CTA. TAT. AAG. TCT. CTA. AAA
Ser-Lys-Gly-Ser-Lys-Thr-Cys-Gly-Phe-Val-Asp-Glu-Arg-Gly-Leu-Tyr-Lys-Ser-Leu-Lys


GGC. GCA. TGC. AAA. CTC. AAG. TTG. TGT. GGA. GTT. CGT. GGA. CTT. AGA. CTT. ATG. GAC. GGA. ACA. TGG
Gly-Ala-Cys-Lys-Leu-Lys-Leu-Cys-Gly-Val-Arg-Gly-Leu-Arg-Leu-Met-Asp-Gly-Thr-Trp


GTC. GCG. ATG. CAG. ACA. TCA. AAT. GAG. ACC. AAA. TGG. TGT. CCT. CCC. GAT. CAG. TTG. GTT. AAT. CTG
Val-Ala-Met-Gln-Thr-Ser-Asn-Glu-Thr-Lys-Trp-Cys-Pro-Pro-Asp-Gln-Leu-Val-Asn-Leu


CAC. GAC. CTT. CGC. TCA. GAT. GAA. ATC. GAG. CAT. CTT. GTT. ATA. GAG. GAG. TTG. GTC. AAG. AAA. AGA
His-Asp-Leu-Arg-Ser-Asp-Glu-Ile-Glu-His-Leu-Val-Ile-Glu-Glu-Leu-Val-Lys-Lys-Arg


GAG. GAG. TGT. CTG. GAT. GCA. TTA. GAG. TCC. ATC. ATA. ACC. ACC. AAG. TCA. GTG. AGT. TTC. AGA. CGT
Glu-Glu-Cys-Leu-Asp-Ala-Leu-Glu-Ser-Ile-Ile-Thr-Thr-Lys-Ser-Val-Ser-Phe-Arg-Arg


CTC. AGT. TAT. TTA. AGA. AAA. CTT. GTC. CCC. GGG. TTC. GGA. AAA. GCA. TAT. ACC. ATA. TTC. AAC. AAG
Leu-Ser-Tyr-Leu-Arg-Lys-Leu-Val-Pro-Gly-Phe-Gly-Lys-Ala-Tyr-Thr-Ile-Phe-Asn-Lys


ACC. TTG. ATG. GAG. GCT. GAA. GCT. CAC. TAC. AAG. TCA. GTC. AGG. ACT. TGG. AAT. GAG. ATC. ATC. CCC
Thr-Leu-Met-Glu-Ala-Glu-Ala-His-Tyr-Lys-Ser-Val-Arg-Thr-Trp-Asn-Glu-Ile-Ile-Pro


TCA. AAA. GGA. TGT. TTG. AGA. GTT. GGA. GGG. AGG. TGT. CAT. CCT. CAT. GTA. AAC. GGG. GTG. TTT. TTC
Ser-Lys-Gly-Cys-Leu-Arg-Val-Gly-Gly-Arg-Cys-His-Pro-His-Val-Asn-Gly-Val-Phe-Phe
```

F i g. 1 (b)

```
AAT. GGT. ATA. ATA. TTA. GGG. CCT. GAC. GGT. CAT. GTT. TTA. ATC. CCA. GAG. ATG. CAA. TCA. TCC. CTC
Asn-Gly-Ile-Ile-Leu-Gly-Pro-Asp-Gly-His-Val-Leu-Ile-Pro-Glu-Met-Gln-Ser-Ser-Leu

CTC. CAG. CAA. CAT. ATA. GAG. TTA. TTG. GAA. TCC. TCA. GTT. ATT. CCC. CTG. ATG. CAC. CCC. CTT. GCA
Leu-Gln-Gln-His-Ile-Glu-Leu-Leu-Glu-Ser-Ser-Val-Ile-Pro-Leu-Met-His-Pro-Leu-Ala

GAC. CCG. TTC. ACA. GTT. TTC. AAG. GAC. GGC. GAT. GAG. ACT. GAG. GAT. TTT. ATA. GAA. GTT. CAC. CTT
Asp-Pro-Phe-Thr-Val-Phe-Lys-Asp-Gly-Asp-Glu-Thr-Glu-Asp-Phe-Ile-Glu-Val-His-Leu

CCC. GAT. GTG. CAC. GAA. CAA. GTC. TCA. GGG. GTT. GAC. CTG. GGT. CTC. CCG. AAC. TGG. GGG. GAG. TAT
Pro-Asp-Val-His-Glu-Gln-Val-Ser-Gly-Val-Asp-Leu-Gly-Leu-Pro-Asn-Trp-Gly-Glu-Tyr

GTA. TTA. CTA. AGT. GCA. GGG. ACC. TTG. ATT. GCC. TTG. ATG. TTG. ATA. ATT. TTC. CTA. ATG. ACA. TGT
Val-Leu-Leu-Ser-Ala-Gly-Thr-Leu-Ile-Ala-Leu-Met-Leu-Ile-Ile-Phe-Leu-Met-Thr-Cys

TGT. AGA. AAA. GTC. GAT. CGG. CCA. GAA. TCT. ACA. CAA. CGC. AGT. CTC. AGA. GGG. ACA. GGA. AGG. AAT
Cys-Arg-Lys-Val-Asp-Arg-Pro-Glu-Ser-Thr-Gln-Arg-Ser-Leu-Arg-Gly-Thr-Gly-Arg-Asn

GTG. TCA. GTC. ACC. TCC. CAA. AGC. GGG. AAA. TTC. ATA. CCT. TCA. TGG. GAG. TCG. TAT. AAA. AGT. GGG
Val-Ser-Val-Thr-Ser-Gln-Ser-Gly-Lys-Phe-Ile-Pro-Ser-Trp-Glu-Ser-Tyr-Lys-Ser-Gly

GGT. GAG. ACT. GGA. CTG. TGA. AGA. TTT. GTC. ATC. TTT. TCG. ACG. CTT. CAA. GTT. CTG. AAG. ATA. ACC
Gly-Glu-Thr-Gly-Leu-***

TTC. CCT. CTA. AGC. TGG. GGG. GAA. TCT. CTG. AGT. TCA. ATA. GTC. CTC. CTT. GAA. CTC. CAT. TCA. ACA

GGG. TGG. ATT. CAA. AAG. TCA. TGA. GAC. TTT. CAT. TAA. TCA. TCT. CAG. TTG. ATC. AAA. CAA. GGT. CAT

GTA. GAT. TCT. CAT. AAT. TCG. GGA. AAT. CTT. CTA. GTA. GTT. TCA. GTG. ACC. GAC. AGT. GCT. TTC. ATT

CCC. CAG. GAA. CTG. ATG. TCA. AAG. GTT. GTT. GAC. GGG. TCA. AGA. GGT. ATT. TCT. GAT. GAC. TCC. GTG

CGT. GGG. CCC. GGA. CAG. AGG. TCA. TAG. TAC. GTC. CCA. TGA. TAG. CGG. ACT. CAG. CAT. GAG. TCG. ATT

GAG. AAA. AGT. AAT. CTG. CCT. CCC. ATG. AAG. GAC. ACC. GGC. AAT. AAC. TCA. CAA. TCA. TCT. TGC. ATC

TCA. GCG. AAG. TGT. GCA. TAA. TTA. TAA. AGG. GGC. TAG. ATC. ATC. TAA. GCT. TTT. CAG. TTG. AGA. AAA

AAA. AAA. AAA. AAA. AAA. AAA. AAA.
```

### G - c D N A

```
┌─32─┐
AAA⋯AAAGCAAACTATTAACATCCCTCAAAAGACTTAGGGAAAGATGGTTCCGCAA───────────────
```

Digestion with unclease

Addition of pSal I linker

| | |
|---|---|
| N9-4 | AGGGAAAGATGGTTCCGCAA──────────── |
| N9-13 | GACTTAGGGAAAGATGGTTCCGCAA──────────── |
| N9-18 | ATGGTTCCGCAA──────────── |
| N9-32 | GGTTCCGCAA──────────── |
| N9-46 | GGGAAAGATGGTTCCGCAA──────────── |

EP 0 324 157 A1

F i g. 3

F i g.  4

Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 140 762 (TRANSGENE S.A.) <br> * Whole document * <br> --- | 1-10 | C 12 N 15/00 <br> A 61 K 39/205 <br> C 12 P 21/00 |
| X | EP-A-0 117 657 (GENENTECH) <br> * Page 9, line 4 - page 11, line 6; claims * <br> --- | 1,2 | |
| X | FR-A-2 515 685 (THE WISTAR INSTITUTE) <br> * Claim * <br> --- | 1,2 | |
| X | PROC. NATL. ACAD. SCI. USA, vol. 81, November 1984, pages 7194-7198; T.J. WIKTOR et al.: "Protection from rabies by a vaccinia virus recombinant containing the rabies virus glycoprotein gene" <br> * Page 7195, column 2, lines 44-65; page 7196, figures 1C,1D; page 7195, column 2, line 50 - page 7197, column 1, line 10 * <br> --- | 3,4,7 | |
| A | NATURE, vol. 312, 8th November 1984, pages 163-166; M.P. KIENY et al.: "Expression of rabies virus glycoprotein from a recombinant vaccinia virus" <br> --- | | TECHNICAL FIELDS SEARCHED (Int. Cl.4) <br><br> C 12 N <br> A 61 K |
| A | NATURE, vol. 294, 19th November 1981, pages 275-278, Macmillan Journals, LTD; A. ANILIONIS et al.: "Structure of the glycoprotein gene in rabies virus" <br> --- | | |
| A,D | NATURE, vol. 219, no. 4585, 11th February 1983, pages 614-620, E. YELVERTON et al.: "Rabies virus glycoprotein analogs: Biosynthesis in Escherichia coli" <br> ----- | | |

The present search report has been drawn up for all claims

| Place of search | Date of completion of the search | Examiner |
|---|---|---|
| THE HAGUE | 24-04-1989 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                           

& : member of the same patent family, corresponding document

EPO FORM 1503 03.82 (P0401)